(19) Europäisches Patentamt
European Patent Office
Office européen des brevets

(11) **EP 1 394 535 B1**

(12) **EUROPEAN PATENT SPECIFICATION**

(45) Date of publication and mention
of the grant of the patent:
**27.08.2008 Bulletin 2008/35**

(51) Int Cl.:
*G01N 27/327* (2006.01)   *C12Q 1/00* (2006.01)

(21) Application number: **03077709.8**

(22) Date of filing: **28.03.2001**

(54) **Meter with rapid response glucose sensor**

Zeitmesser mit schnell ansprechenden Glucose Sensor

Compteur de temps avec capteur de glucose à réponse rapide

(84) Designated Contracting States:
**AT BE CH CY DE DK ES FI FR GB GR IE IT LI LU MC NL PT SE TR**

(30) Priority: **28.03.2000 US 537065**
**28.03.2000 GB 0007522**

(43) Date of publication of application:
**03.03.2004 Bulletin 2004/10**

(62) Document number(s) of the earlier application(s) in accordance with Art. 76 EPC:
**01924446.6 / 1 269 173**

(73) Proprietor: **Diabetes Diagnostics, Inc.**
**Waltham, MA 02453 (US)**

(72) Inventors:
• **Davies, Oliver William Hardwicke**
**Inverness IV2 5PG,**
**Scotland (GB)**
• **Beckingham, Helen Elizabeth**
**Inverness IV2 5PG,**
**Scotland (GB)**
• **Hall, Geoffrey F.**
**Fortrose IV10 85H,**
**Scotland (GB)**

(74) Representative: **Mercer, Christopher Paul et al**
**Carpmaels & Ransford**
**43, Bloomsbury Square**
**London WC1A 2RA (GB)**

(56) References cited:
**EP-A- 0 859 230**      **WO-A-00/73778**
**WO-A-98/35225**

## Description

Field of the Invention

This application relates to a disposable electrochemical glucose sensor of the type used by diabetics to monitor blood glucose levels.

Background of the Invention

Disposable strip electrochemical glucose sensors have been commercially available for over 10 years, and are described in various patents including US Patents Nos. 4,711,245, 5,708,247 and 5,802,551. These sensors utilize redox mediators to facilitate charge exchange between enzyme and electrode. These devices offer significant advantages over the older optical technology, such as the fact that the blood does not go into the meter and the meters themselves tend to be much lighter and less cumbersome; but they also suffer some disadvantages. The electrochemical tests results are typically affected by other electroactive species present in the sample and also by the oxygen content and haematocrit of the sample.

The reason for the interference by electro-active species is very straight-forward. Species which are readily oxidizable result in an increased current which leads to an elevated reading. The increased current may be due to direct oxidation at the electrode surface or arise *via* redox catalysis. Some manufacturers have tried to address this problem by using an auxiliary electrode to make a background subtraction. While this approach is useful, it adds an extra manufacturing step; adding cost and an extra measurement with its associated errors, thereby degrading precision. Background subtraction can also lead to an overcorrection since the efficiencies of interferant redox catalysis can be different on the two electrodes depending on the analyte concentration.

The oxygen and haematocrit effects are linked. Oxygen is the natural cofactor for glucose oxidase, so in the presence of oxygen there will be strong competition between oxygen and redox mediator resulting in a depressed signal. Similarly, since hemoglobin is a highly efficient oxygen delivery medium, high sample haematocrits will also result in depressed signals. Exclusion membranes which keep blood cells away from the electrode surface have been proposed to reduce the haematocrit effect (US Pat. No. 5,658,444). This approach adds additional manufacturing steps, and is in any event only effective for a part of the oxygen-based effect.

Thus, there remains a need for a disposable electrochemical devices which provide readings for blood analyte levels, particularly glucose, that are at most minimally impacted by the presence of interferants.

Summary of the Invention

In accordance with the invention, a disposable electrochemical sensor for the detection of an analyte such as glucose in a liquid sample is provided. The sensor comprises a working electrode and a reference electrode disposed within a sample-receiving cavity, a reagent layer disposed within the sample-receiving cavity and over the working electrode, said reagent layer comprising at least an enzyme for producing an electrochemical signal in the presence of the analyte, wherein the sample-receiving cavity has a volume of less than 1.5 $\mu$l, and wherein the sensor provides a measurement that correlates sufficiently well (for example $R^2 > 0.95$) with the amount of analyte in a period of 10 seconds or less to allow use the measurement in the precise and accurate detection and quantitation of the analyte.

The sensor is used in combination with a meter for detection of the analyte in a liquid sample. A suitable meter comprises a timing circuit for controlling the measurement of current indicative of analyte in the sample following detection of sample application to a test strip inserted in the meter, wherein the timing circuit causes the measurement of current to occur at a time 15 seconds or less after the detection of sample application.

Brief Description of the Drawings

Fig. 1 illustrates the diffusional movement of reactant species in the vicinity of a disposable electrode;
Fig. 2 shows a cross sectional view of a biosensor in accordance with a first embodiment of the invention;
Fig. 3 shows a cross sectional view of a biosensor in accordance with a second embodiment of the invention;
Fig. 4 shows an apparatus for web printing of a face-to-face sensor device;
Fig. 5 shows a partially constructed face-to-face sensor device;
Fig. 6 shows a cross-section view of a sensor in accordance with the invention;
Fig.7 shows a plot of correlation coefficient vs test time;
Fig. 8 shows an exterior view of a meter in accordance with the invention;
Figs. 9A-C show the construction of a sensor in accordance with the invention; and
Fig. 10 shows a comparison of a commercial strip with a rapid response strip in accordance with the invention.

Detailed Description of the Invention

The key to improving electrochemical strip performance lies in designing the strip such that the mediated reaction is favoured over the interfering reactions. In the case of glucose detection, the analyte specific reaction is a mediated reaction involving enzymatic generation of reduced mediator followed by oxidation of the mediator at the electrode surface. We therefore concluded that the test should be constructed such that these reactions take place in close proximity with the electrode surface in order to provide the maximum collection efficiency.

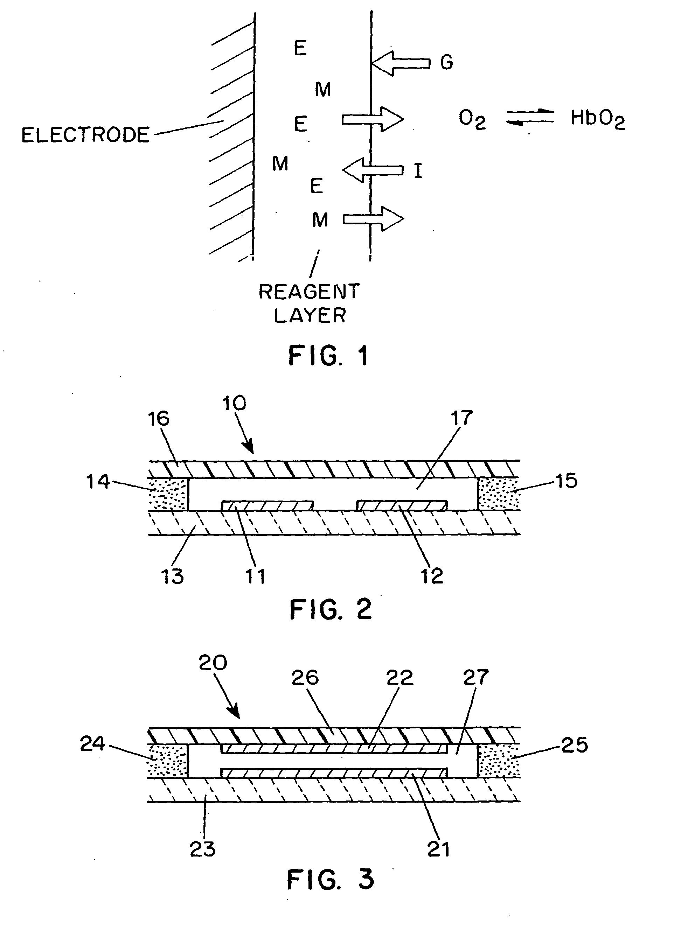

It is worth considering the diffusion processes taking place during a test. Consider the application of a sample to the test strip as shown in Fig.1. The test strip, in it's dry state, includes an electrode coated with a reagent layer containing enzyme, E, and mediator, M. The test sample contains glucose, G, electrochemical interferants, I, and oxygen, $O_2$, which may be bound to hemoglobin, Hb. On application of the sample there is a net diffusional flux of E and M away from the electrode towards the test sample and a net diffusional flux of G and I towards the electrode. Hence at very short times after sample application most of the enzyme is still close to the electrode and reaction with glucose has a high probability of resulting in generation of a reduced mediator molecule close enough to the electrode to be captured. At longer times, much of the enzyme has diffused "deeper" into the sample and can react with glucose here. This has two effects. Firstly, there is a high probability of the reduced enzyme being oxidized by $O_2$ rather than M, since the concentration of M will diminish further from the electrode and the concentration of $O_2$ will increase further from the electrode (because of this same reaction). Even if the reduced enzyme does react with M, the probability of the reduced M diffusing back to the electrode to be reoxidized with the concomitant production of a detectable signal is low. Secondly, the sequence of reactions just described has the effect of depleting the inwardly diffusing G, so that the amount of G that actually arrives in the vicinity of the electrode where it can be detected with some efficiency is reduced. Clearly both of these factors contribute to a reduced signal in the presence of oxygen in the sample.

Similarly, common interferants are easily oxidized materials such as ascorbate, acetaminophen and uric acid which upon reaching the electrode surface are oxidized along with reduced mediator that may be present. Since this effect can only occur when I is present near the electrode surface, it will be at its minimum at short times before diffusion of I to the electrode has occurred.

As is apparent from this mechanistic explanation, one solution to both of the problems of interferants and hematocrit/oxygen levels is to make the measurement at very short times. An alternative solution is to restrict the sample volume so that the surface area of the electrode is very large compared to the sample volume. A good configuration is one that ensures that the sample layer over the electrode is very thin (e.g. <200 microns). One benefit of limiting the sample volume is that the solution hydrodynamics settle down more rapidly. With a large sample volume convective effects in the sample lead to noise in the measurement. By maintaining a low sample volume in the form of a thin film convective effects are minimized. This means that with a low sample volume it is possible to make measurements earlier.

In practice, these solutions are related and are both implemented in the biosensors of the present invention. Thus, the present invention provides disposable electrochemical sensors and associated meters which are adapted for taking electrochemical measurements of the amount of an analyte in a sample, for example for quantification of blood glucose levels, in a shorter time than previously known systems. The sensors of the invention take advantage of the synergistic relationship between short measurement times and small sample volumes to achieve superior performance. Low sample volume allows earlier measurement because of early settling of hydrodynamic effects, and thus facilitates measurements at short times. Low sample volume also necessitates short time measurements because the small signal diminishes at longer times and therefore cannot provide a reliable reading. By choosing this kind of configuration we ensure that the mediator concentration is kept high so that the mediator competes more effectively with oxygen for the reduced enzyme.

Achieving a device which utilizes a small sample volume is highly desirable from the patient point of view. The challenge is creating a device which utilizes a small sample volume to produce reliable measurements of the analyte concentration. The first part of this process is the definition of a small volume sample-receiving cavity. The volume of this cavity is defined by the area of the electrodes and the thickness of the gap between the electrodes. There is a lower limit to the area of electrodes which can be achieved by any given printing process, determined by edge definition and print tolerances. One way to improve this precision when using known electrode printing inks is with the printing methodology described in commonly assigned International Patent Publication No. WO00/42422.

Once the "area" of the electrodes has been minimized, the sample volume is further defined by the gap between the electrode surfaces. The primary goal is a thin, but consistent gap. It should be remembered, however, that if a low sample volume is achieved by using a very thin gap (i.e. < 200 $\mu$m), the usual conditions of semi-infinite diffusion are not met. Because of this, the diffusion layer can extend across the entire gap, and significantly deplete the sample. Under these circumstances, the precision of the devices becomes influenced by the additional factor of the precision of the assembly process that determines the gap size. There is a relationship between the measurement time and the size at which precision in the gap size becomes important, which can be understood from consideration of the formula

$$L = \sqrt{(Dt)}$$

where L is the diffusion length, D is the diffusion coefficient and t is time. When the test time is reduced from 15 seconds to 5 seconds, the diffusion length is reduced by a factor of 3. What this means in practical terms is that by shortening the measurement time, one can reduce the size of the gap further, without running into the limiting condition where precision in the gap becomes a substantial factor in the precision of the device. Thus, for example, assuming a diffusion coefficient of $10^{-5}$ cm$^2$sec$^{-1}$ a 5 second test would require a gap greater than 70 $\mu$m, compared to 125 $\mu$m which would be required for a 15 second test. Considering these factors, a suitable configuration for a sensor in accordance with the invention has a sample receiving cavity with a volume of less than 1.5 $\mu$l. Combined with considerations about the gap size, this means that the working electrode is desirably sized such that the ratio of the surface area of the working electrode to the gap size is about 0.5 to 100mm. In a specific preferred configuration, the area of each electrode is 0.8 mm$^2$ and the gap is 100-150 $\mu$m, to define a sample-receiving compartment with a volume of 0.5 to 0.8 $\mu$l.

Fig. 2 shows an electrochemical sensor 10 in accordance with a first embodiment of the invention. Electrodes 11 and 12 are formed on a base substrate 13. The base substrate 13 in combination with spacers 14,15 and top cover 16 define a cavity 17 in which the electrochemical reactions occur. In an exemplary embodiment, the electrodes have a surface area of 5 mm$^2$ and the volume of the cavity is suitably less than 1.5 $\mu$l, preferably less than 1 $\mu$l and most preferably less than 0.5 $\mu$l.

A device of the type shown in Fig. 2 can be manufactured as follows. Electrodes 11 and 12 are deposited onto substrate 13. The specific manner of depositing will be determined by the nature of electrodes, although screen printing is a preferred technique for many materials. The area of the electrode which will be exposed to sample in the chamber is defined by depositing an insulating mask over the electrodes. (See commonly assigned International Patent Publication No. WO00/42422). Next, the reagent layer is deposited. This layer may cover both electrodes, or it may be confined to the area over the working electrode. Spacers 14 and 15 are then formed in a pattern around the electrodes. In a preferred embodiment, these spacers are formed by printing a layer of adhesive having a dry height of about 150 $\mu$m. This spacer defines the capillary gap without the need to utilize a preformed solid material and thus substantially facilitates the production of the devices of the invention. The final step is the application of a cover 16 to complete the chamber 17. In the preferred embodiment, the cover 16 is affixed to the device via the adhesive spacers 14,15.

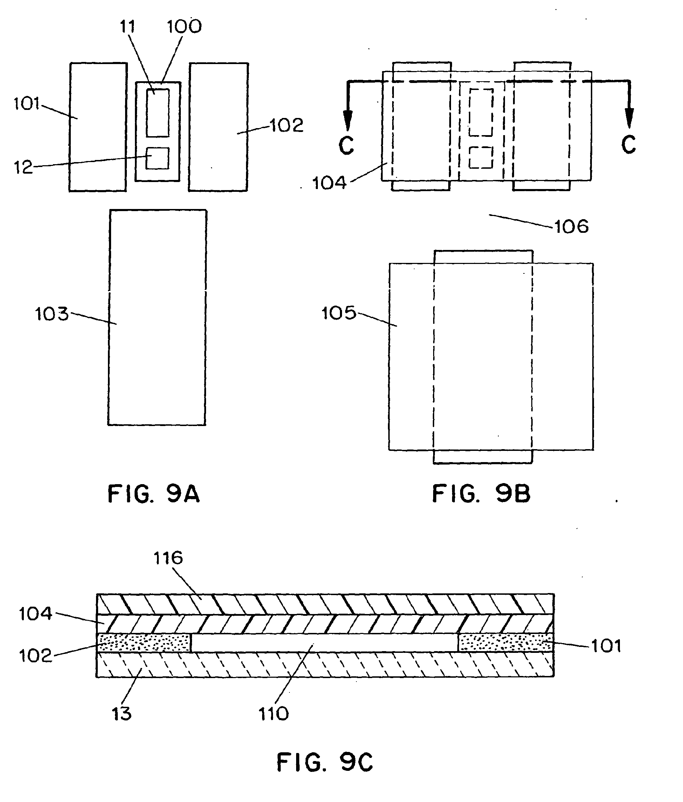

Figs. 9 A-C illustrate a specific embodiment of a manufacturing technique for the production of a sensor in accordance with the invention. The figure shows a single sensor, but it will be appreciated that more than one sensor will generally be prepared. Fig. 9A shows the structure of the device before lamination of the cover. The sensor at this stage has two electrodes 11,12 deposited on a substrate (not shown for clarity). Electrical connections to these electrodes are not shown. A reagent pad 100, for example containing an appropriate enzyme for the analyte, is deposited over both electrodes. Adhesive pads 101, 102 and 103 are deposited on three sides of the reagent pad. Two pieces 104, 105 of a hydrophilic film (such as 3M 9962, a 100 micron thick surfactant-treated optically clear polyester film) are then placed in two locations, one spanning the adhesive pads 101 and 102 and covering the electrodes and reagent pad, and one covering a portion of the adhesive pad 103 to provide a support of consistent height for receiving the cover 116. (Fig. 9B) The positions of these pieces of hydrophobic film creates a capillary chamber over the two electrodes. The hydrophilic coating of the film encourages the movement, by capillary action, of the test liquid into the sample chamber created. The gap 106, formed in the area where there is no spacer or film allows air to escape from the back of the chamber as the test liquid moves into the sample chamber created. A pressure sensitive tape is applied as a top cover 116 over the hydrophilic films. The top cover 116 is suitably formed of a polyester film and can be ocated with either a heat-activated adhesive or a pressure sensitive adhesive. The final step is cutting the device to create the appropriate opening sample chamber, for example by cutting along the dashed line C-C in Fig. 9B. Fig. 9C shows an end view of the device after being cut along this line C-C. As shown, the capillary entrance 110 to the sample chamber is defined by the substrate 13, the adhesive pads 101, 102 and the hydrophilic film 104 and the top cover 116. The films 104 and 105 are supported by the adhesive pads 101 and 102.

Fig. 3 shows an electrochemical sensor 20 in accordance with a second embodiment of the invention. Electrodes 21 and 22 are formed respectively on a base substrate 23 and a top cover 26. The base substrate 23 in combination with spacers 24, 25 and top cover 26 define a cavity 27 in which the electrochemical reactions occur. The sensor is constructed with a low volume and thin gap between the base substrate 23 and the top cover 26, for example from 50 to 200 mu m. It should be noted that the surface area of the electrodes can double for the same size device, because of the folded, face-to-face configuration.

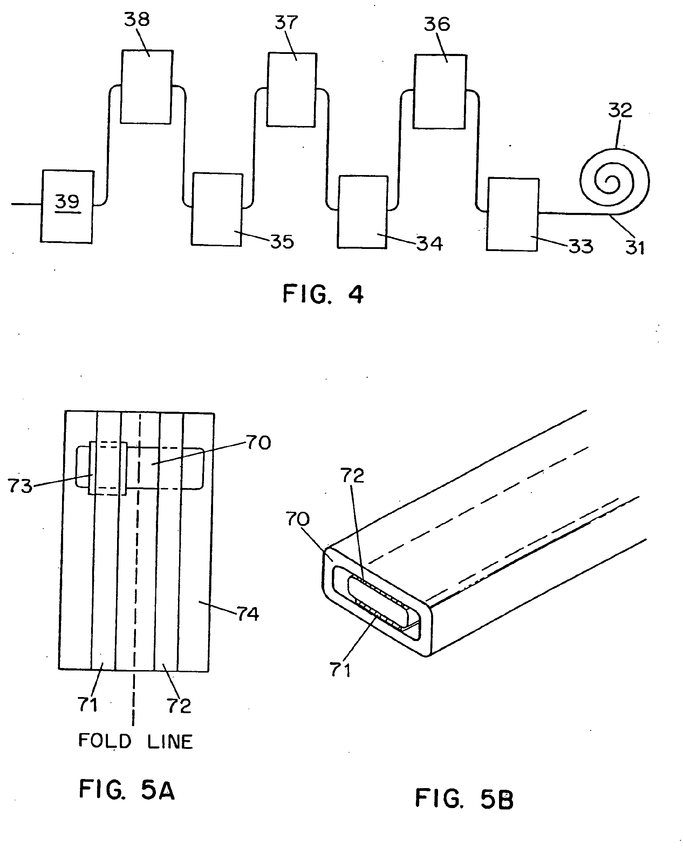

A device with this structure can be made using web printing technology. This technology utilizes an apparatus of the type shown schematically in Fig. 4. A running web of substrate 31 is provided on a feed roll 32 and is transported over a plurality of print stations 33, 34, and 35, each of which prints a different layer onto the substrate. The number of print stations can be any number and will depend on the number of layers required for the particular device being manufactured. Between successive print stations, the web is preferably transported through a dryer 36, 37, and 38, to dry each layer before proceeding to the deposition of the next. After, the final dryer 38, the printed web is collected on a take up roll or introduced directly into a post-processing apparatus 39. To make a device with the structure shown in Fig. 3 in this apparatus, parallel conductive tracks 71 and 72; reagent layer(s) 73 and an insulation layer 74 are deposit on a substrate 70 as shown in Fig. 5. The substrate is then folded along a fold line disposed between the two conductive tracks to produce a sensor in which two face-to-face electrodes are separated by a reagent layer. An electrode geometry with the electrodes disposed on opposing surfaces within the cavity is beneficial because the voltage drop due to solution resistance is low as a result of the thin layer of solution separating the electrodes.

In each of the embodiments of the invention described above, the cavity is defined by insulative materials. Suitable insulative materials for this purpose include nylon, polyester, polycarbonate and polyvinylchloride. Suitable materials for use as the substrate include polyester films, for example a 300 micron polyester fim, and other insulating substrate materials such as polyvinyl chloride (PVC) and polycarbonate. A specific polyester-based printable dielectric material from which the insulating mask ca be formed is ERCON R488-B(HV)-B2 Blue. Within the cavity, a working and a reference electrode are formed from a conductive material. Suitable conductive materials include conductive carbon, gold, platinum, aluminum or doped semiconductor materials such as n-type $SnO_2$. Preferred conductive carbon materials are ERCON ERC1, ERCON ERC2 and Acheson Carbon Electrodag 423. Carbon with these specification is available from Ercon, Inc. (Waltham, Massachusetts, USA), or Acheson Colloids (Princes Rock, Plymouth, England). Semiconductor electrodes offer an attractive option because they can be functionalized to permit the surface attachment of enzymes or other components of the reagent layer. This provides the benefits associated with immobilization, and also permits direct electron transfer between the reagent and the electrode.

The electrodes may be made from different materials or may be of the same material. Embodiments in which the electrodes are of the same composition, for example a carbon-electrode, can offer advantages. Specifically, the use of a single electrode material allows the working and the reference electrodes to be deposited in a single step, thus eliminating an electrode print from the production process. The two electrodes can be printed very close together since the separation between is determined solely by the artwork on one screen (tolerance about 200 $\mu$m) and not on the alignment which can be achieved between separate print runs (tolerance over 0.5 mm) This allows the reaction area to be more compact and thus leads to a reduction in the volume of blood required to cover the electrodes.

The working electrode has one or more reagent layers disposed over the electrode which contain the enzyme and mediator used in the detection of the target analyte. Thus, for example, in a glucose sensor, the reagent layer(s) would include an enzyme such as glucose oxidase and a mediator such as ferricyanide, metallocene compounds, quinones, phenazinium salts, redox indicator DCPIP, and imidazole-substituted osmium compounds. The reagent layer may be a single layer including both enzyme and mediator, or may be constituted from a plurality of sub layers, some containing enzyme or enzyme and mediator and some containing only mediator.

Because the devices of the invention are intended to be used at short time intervals, an important characteristic of the electrodes is the ability to rapidly hydrate. Hydration rate is determined by the reagent layer composition. An electrode system which utilizes a silica-based reagent layer of the type described in US Patent No. 5,708,247, and International Patent Publication No. WO00/42422 permits rapid wetting and hydration and it therefore suitable for use in the sensors of the invention. The optimal material for the reagent layers of the electrodes of the sensors of the invention is one which hydrates rapidly to form a gel which remains in contact with the electrode surface and retains reagents in the vicinity of the electrode. If the reagent layer disperses rapidly following hydration, the reagents (and in particular the enzyme reagent) are rapidly lost from the vicinity of the electrode surface where they are most beneficial for the development of a signal reflecting analyte concentration in a sample.

The reagent layer must also comprise a mediator in a form available for immediate participation in the generation of signal reflecting analyte concentration. In the case of an analyte such as glucose which is oxidized by the enzyme, this means that mediator must be rapidly soluble and present in the oxidized form. In a commercial glucose strip sold by Medisense under the tradenames QID™ and EXACTECH™, the mediator is actually present in the reduced form and must be oxidized *in situ* before is can be can participate in a glucose monitoring reaction. This limits the response time of the strip, and precludes its use at short test times.

In the case of the reference electrode, the electrode needs to be rapidly hydrating, and also able to stabilize quickly enough to source the current demanded by the working electrode instantaneously, i.e, within 200 msec of hydration. A conventional silver/silver chloride reference electrode does not stabilize quickly enough. A ferri-ferrocyanide reference on the other hand can be made to equilibrate very rapidly. In this design, a mediator-containing layer is used that solubilizes or disperses rapidly. In a specific embodiment of the invention, carbon ink electrodes are used with a reagent layer containing potassium ferricyanide as the mediator. Glucose oxidase is used as the enzyme in a hydroxy ethylcellulose-silica base with polymers added to increase the hydrophilic nature of the formulation. This system has a very high surface area and wets very rapidly.

In addition to the working electrode and the reference electrode, the device of the invention may be constructed to include a third electrode. The third electrode may be a dummy electrode, intended to compensate for background reactions, or a counter electrode of a conventional three electrode system. The third electrode might also be an identical working electrode.

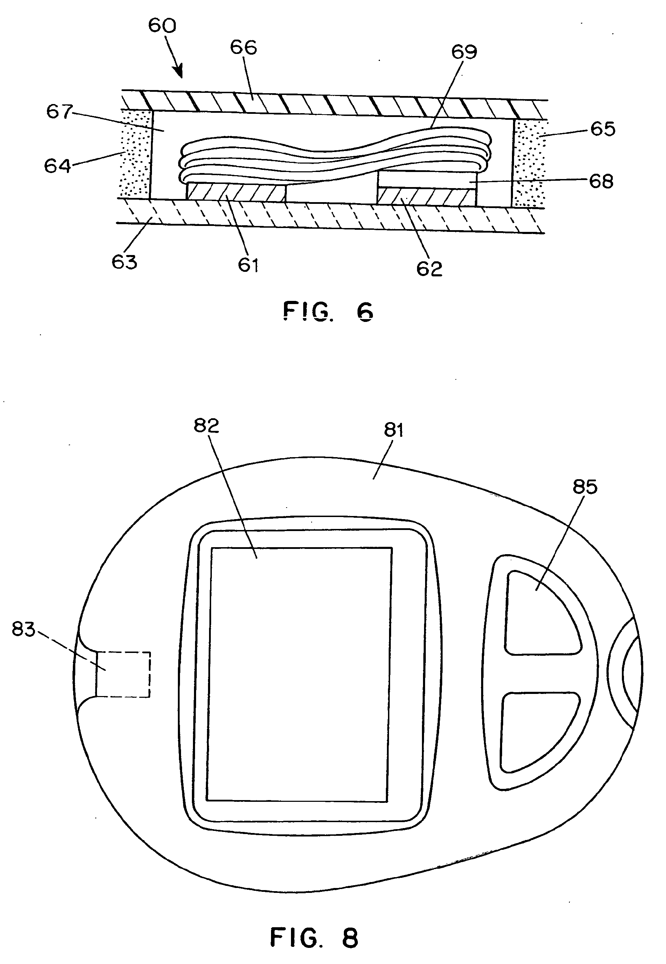

In the embodiments of the invention discussed above, all of the layers are rapidly solubilized or hydrated. While rapid solubilization or at least hydration of the oxidized mediator is not a problem for interferant consumption, and possibly helps achieve this requirement, it is not entirely a good characteristic for an enzyme-containing layer, as described earlier, since this facilitates the enzyme diffusing away from the area close to the electrode where it is most beneficial. A useful configuration that combines both aspects, therefore, is shown in Fig 6. In this embodiment of the invention, the sensor 60 has a cavity 67 formed from a bottom substrate 63, spacers 64, 65 and a top cover 66. Two carbon electrodes 61, 62 are disposed on the bottom substrate 63 within the cavity 67. Electrode 62 is coated with a relatively thin (e.g. 5 $\mu$m) viscous gel layer 68 containing enzyme and mediator. Both electrodes 61, 62 are then covered with a relatively thick (e.g. 25 $\mu$m) dispersion layer 69 containing mediator, but no enzyme.

In another embodiment of the invention, two separate layers are configured to further reduce the effects of interferants. One way to capitalize on the chemical consumption of interferants is to provide a reagent layer with an excess of oxidized mediator on the outside. In a particularly attractive configuration an electrode is coated with a thin reagent layer containing enzyme and mediator and then a thick layer containing only mediator. Both layers are deposited in a matrix which limits diffusion but which is rapidly hydrated so that it can carry a current. By confining the enzyme to a thin layer the enzyme is largely held in close proximity with the electrode so that the parasitic reactions described above are unimportant. The thick outer mediator layer provides a barrier to inward diffusing interferants and remains in the desired position because of the diffusion-limiting matrix. An optional third layer may be included outside the first and second layers containing mediator in a rapidly hydrated dispersable matrix. Once again, by ensuring that the sample volume is small, the *total amount* of interferant in the sample is kept to a minimum, and the *concentration* of oxidized mediator on re-constitution is high so that the mediator effectively removes interferant. Obviously, at longer times the local concentration of mediator will fall as it diffuses out into the sample and interference will become more significant. In our experience a sample volume of less than 1 $\mu$l, preferably 0.5 $\mu$l, is ideal.

Sensors made in accordance with the invention allow the taking of test measurements in much shorter times than achieved using known sensors. By shortening the test time, haematocrit effects can be reduced. If the sensor comprises an electrode covered with a reagent layer which has a retarding effect on certain blood components such as white cells and erythrocytes, then at short times the fluid arriving at the electrode will contain significantly fewer of these components than at long times.

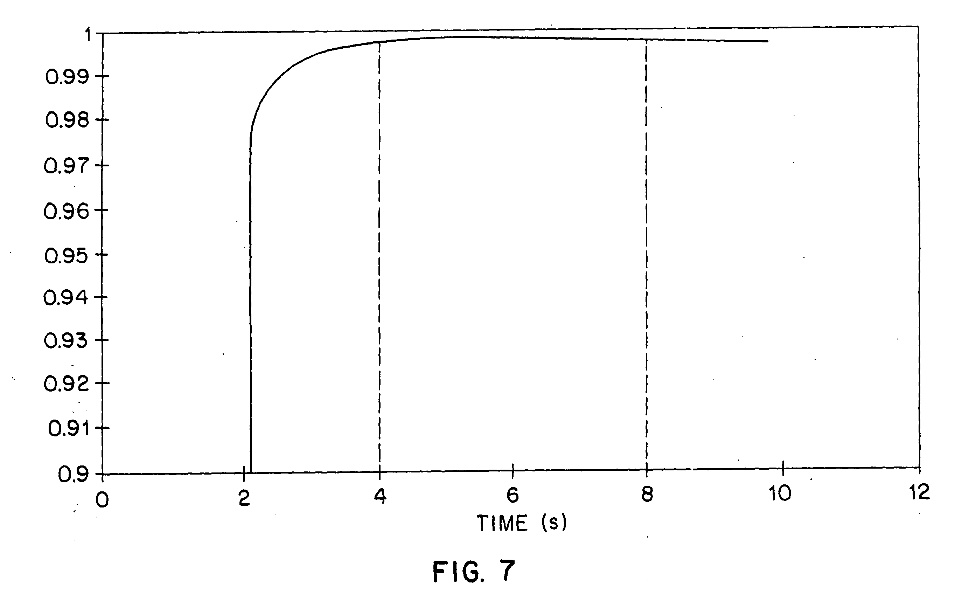

Fig.7 shows a plot of correlation coefficient versus test time. At extremely short test times correlation is poor because the system has not yet stabilized. At very long test times the correlation also starts to degrade. Given the objective of limiting interferences by shortening the test time, the test will suitably be conducted in the regime indicated by the dashed lines, which for the sensors described below will be less than 10 seconds and preferably around 5 seconds. The disposable sensors of the invention work in combination with a test meter to provide accurate measurements of glucose within this time regime. Thus, the sensor is configured to provide signals which provide accurate and reliable information at short times, and the meter into which the sensor is inserted is adapted to collect information during this time.

Fig. 8 shows an exterior view of an exemplary hand-held meter in accordance with the invention. Like conventional meters, the meter of the invention has a housing 81 with a display 82 for displaying the results, and a slot 83 for insertion of the disposable sensor. Buttons 85 and/or switches may be included for operation of the meter, including recall of stored results, calibration checks and the like. Where the meter of the invention differs from the conventional meter is the in electronics within the housing. In the conventional meter, the addition of a liquid sample, such as a drop of blood, to a disposable sensor in the housing starts a measurement cycle during which reagents are dissolved and a reading taken. The start of the cycle may also be triggered by the depression of a button by the user, although this is not preferred. The microprocessor in a meter is typically in a "sleep" mode and "wakes up" periodically (for example every ½ second) to check interrupts. If the program detects that an interrupt flag is set, indicating that a strip has been inserted in the meter or the start button has been pressed, the program enters RUN mode. In this mode, typically a potential is applied to the strip and the microprocessor monitors the output (duty cycle) of a pulse-width monitor which indicates the level of any current drawn by the strip. As soon as the sample is applied to the strip, a current flows since the strip is already subject ed to a polarization potential. Detection of this start current initiates a timing sequence. Timing is controlled by the microprocessor. There are two crystals: a 4 MHz clock for operational function (i.e., performing measurements) and a 32 mHz clock which keeps time in the Off mode. On initiation of the timing process, the applied potential may either (1) be maintained at a constant level or (2) be varied following a predetermined profile. In either case, the current is measured after a predetermined time to assess the amount of analyte in the sample. By way of example, the data shown in Fig. 7 was collected in a system in which the sample application was detected at t=0, the applied potential was removed for 2 sec, during which time the strip is an open circuit, and then the same potential reapplied. The current was measured at numerous time points and the correlation of current with analyte concentration determined at each time point.

In commercially available meters known in the art, the measurement cycle is established to make the current measurement at 20 to 60 seconds after the detection of sample. In the meters of the invention, which are particularly adapted for use with rapid-response strips of the invention, the measurement cycle is established to make current measurements at a time 15 seconds or less after the detection of sample, and preferably at a time from 5 to 10 seconds after the detection of sample.

The invention will now be further described with reference to the following non-limiting examples.

Example 1

Rapid response glucose sensors in accordance with the invention were prepared using the procedures outlined in Figs 9A-C and the following materials:

  substrate: polyester film
  carbon ink formulation: Ercon conductive carbon
  reagent layer composition: as described below
  adhesive: water-based acrylic copolymer adhesive (Apollo Adhesives)
  hydrophilic film: 3M 100 micron hydrophilic film 9962
  top cover: pressure sensitive adhesive coated polyester strip (Tape Specialities)

The reagent layer was formulated as follows. 100 ml of 100 mM aqueous trisodium citrate was adjusted to pH 5 by the addition of 1 M citric acid. To this 5 g of hydroxyethylcellulose (HEC), 1 g polyvinyl alcohol,1 g PVP-VA S-630 poly(vinyl pyrrolidone vinyl acetate), and 0.5 ml of DC 1500 Dow Corning anti-foram were added and mixed by homogenization. The mixture was allowed to stand overnight to allow air bubbles to disperse and then used as a stock solution for the formulation of the coating composition. 7.5 g of Cab-o-Sil TS610 were gradually added by hand to the HEC solution until about 4/5 of the total amount was added. The remainder was added with mixing by homogenization. The mixture was then rolled for 12 hours. 11 g of potassium ferricyanide was then added and mixed by homogenization until completely dissolved. Finally, 2.8 g of glucose oxidase enzyme prepartion (250 Units/mg) was added and then thoroughly mixed into the solution. The resulting formulation was ready for printing, or could be stored with refrigeration.
The sensors were used to test standard glucose solutions and the current measured at different times following addition of the glucose to the sensor. The correlation coefficient between the actual glucose concentration and the measured glucose concentration was determined for each time interval. Fig. 7 shows a plot of the results. As shown, the correlation coefficient has achieved a maximum and high value by 5 seconds after the addition of glucose to the sensor.

Example 2

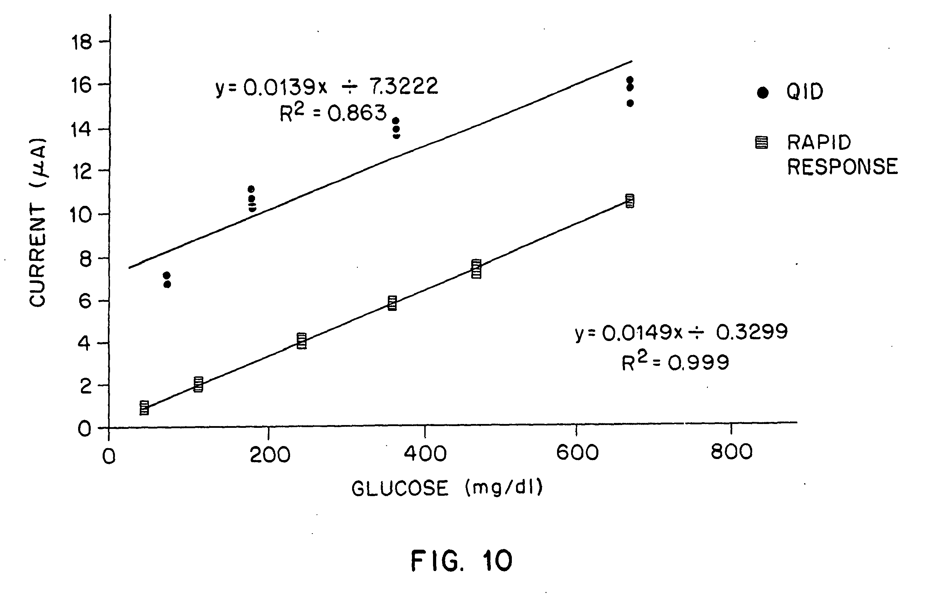

Rapid response glucose sensors in accordance with the invention were prepared as in Example 1. These sensors were utilized to determine the amount of current at five seconds after exposure to different concentrations of glucose. For comparison, a Medisense QID glucose sensor was tested under the same conditions. Fig. 10 shows the results of this experiment graphically. As shown, the linearity of the response of the rapid response sensor in accordance with the invention is very good ($R^2$=0.999). The linearity of the QID sensor at five seconds was not as good ($R^2$=0.863).

**Claims**

1. A meter comprising:

  a housing (81);
  a display (82) for displaying results;
  a slot for insertion of a disposable electrochemical sensor for detection and/or quantification of an analyte in a liquid sample; and
  a timing circuit adapted to control measurement of current indicative of analyte in the sample following detection of sample application to the sensor when the sensor is inserted in the meter, **characterised in that** the timing circuit is further adapted to cause the measurement of current to occur at a time 15 seconds or less after the detection of sample application.

2. The meter of claim 1, wherein the timing circuit causes the measurement of current to occur at a time 10 seconds or less after the detection of sample application.

3. The meter of claim 1, wherein the timing circuit causes the measurement of current to occur at a time 5 seconds or less after the detection of sample application.

4. The meter of any of claims 1 to 3, wherein the meter comprises a hand-held housing in which the timing circuit is disposed, said housing having an opening therein for receiving the sensor.

**Patentansprüche**

1. Meßgerät, das aufweist:

  ein Gehäuse (81) ;

eine Anzeige (82) zum Anzeigen von Ergebnissen;

einen Schacht zum Einsetzen eines entsorgbaren elektrochemischen Sensors für das Erfassen und/oder die Mengenbestimmung eines Analyten in einer Flüssigkeitsprobe; und

eine Taktschaltung, die dazu ausgelegt ist, das Messen eines Stromes zu steuern, der für den Analyten in der Probe bezeichnend ist, anschließend an das Erfassen der Probenbeaufschlagung bei dem Sensor, wenn der Sensor in das Meßgerät eingesetzt ist,

**dadurch gekennzeichnet, daß** die Taktschaltung weiter dazu ausgelegt ist, die Messung des Stromes zu einem Zeitpunkt von 15 Sekunden oder weniger nach dem Erfassen der Probenbeaufschlagung hervorzurufen.

2. Meßgerät nach Anspruch 1, bei dem die Taktschaltung bewirkt, daß die Messung des Stromes zu einem Zeitpunkt von 10 Sekunden oder weniger nach dem Erfassen der Probenbeaufschlagung geschieht.

3. Meßgerät nach Anspruch 1, bei dem die Taktschaltung bewirkt, daß die Messung des Stromes zu einem Zeitpunkt von 5 Sekunden oder weniger nach dem Erfassen der Probenbeaufschlagung geschieht.

4. Meßgerät nach einem der Ansprüche 1 bis 3, wobei das Meßgerät ein tragbares Gehäuse aufweist, in dem die Taktschaltung angeordnet ist, wobei das Gehäuse eine Öffnung zum Aufnehmen des Sensors in dieser hat.

**Revendications**

1. Mesureur comprenant :

un boîtier (81) ;
un affichage (82) pour afficher des résultats;
une fente pour l'insertion d'un capteur électrochimique jetable pour la détection et/ou la quantification d'une substance à analyser dans un échantillon liquide ; et
un circuit de comptage de temps adapté pour commander la mesure de courant indicative de substance à analyser dans l'échantillon après détection d'une application d'échantillon au capteur quand le capteur est inséré dans le mesureur,

**caractérisé en ce que** le circuit de comptage de temps est adapté en outre pour forcer la mesure de courant à se produire à un temps de 15 secondes ou moins après la détection d'une application d'échantillon.

2. Mesureur selon la revendication 1, dans lequel le circuit de comptage de temps force la mesure de courant à se produire à un temps de 10 secondes ou moins après la détection d'une application d'échantillon.

3. Mesureur selon la revendication 1, dans lequel le circuit de comptage de temps force la mesure de courant à se produire à un temps de 5 secondes ou moins après la détection d'une application d'échantillon.

4. Mesureur selon l'une quelconque des revendications 1 à 3, dans lequel le mesureur comprend un boîtier portatif dans lequel le circuit de comptage de temps est disposé, ledit boîtier ayant une ouverture dans celui-ci pour recevoir le capteur.

FIG. 1

FIG. 2

FIG. 3

FIG. 4

FOLD LINE

FIG. 5A

FIG. 5B

FIG. 6

FIG. 8

FIG. 7

EP 1 394 535 B1

FIG. 9A

FIG. 9B

FIG. 9C

FIG. 10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4711245 A
- US 5708247 A
- US 5802551 A
- US 5658444 A
- WO 0042422 A